# EUROPEAN PATENT SPECIFICATION

(11) **EP 1 782 743 B1**
(45) Date of publication and mention of the grant of the patent: **18.03.2009**
(21) Application number: 06255647.7
(22) Date of filing: 02.11.2006
(51) Int. Cl.: A61B 18/14, A61B 18/08, A61B 17/072

(54) **Electrosurgical stapling instrument with disposable severing/stapling unit**
Elektrochirurgisches Klammerinstrument mit einer wegwerfbaren Abtrenn-/Klammereinheit
Agrafeuse électrochirurgicale avec une unité pour agrafer et séparer jetable

(30) Priority: 04.11.2005 US 268290
(43) Date of publication of application: 09.05.2007
(73) Proprietor: ETHICON ENDO-SURGERY, INC., Cincinnati, Ohio 45242 (US)
(72) Inventor: Shelton IV, Frederick E., Hillsboro, Ohio (US)
(74) Representative: Tunstall, Christopher Stephen

(56) References cited:
- EP-A- 0 705 571
- EP-A1- 0 888 749
- WO-A-03/057058
- US-A- 5 833 690

## Description

### FIELD OF THE INVENTION

The invention generally relates to surgical instruments. The invention more particularly relates to transmission and application of electrical energy in association with the use of surgical instruments to promote closure and healing of severed and stapled tissue.

### BACKGROUND

Conventional surgical staplers that can be used to simultaneously make incisions in tissue and apply lines of staples on opposing sides of the incisions are known in the art. Such devices commonly include a pair of cooperating jaw members that, when employed in endoscopic or laparoscopic applications, are capable of passing through a cannula passageway. One of the jaw members typically has a staple cartridge having at least two laterally spaced rows of staples. The other jaw member defines an anvil having staple-forming pockets correspondingly aligned with the rows of staples in the staple cartridge. In addition, a knife or other cutting edge is included in the surgical stapler which is designed to cut tissue during a surgical procedure.

In operation of the surgical stapler, a clinician can close or clamp the jaw members to position tissue prior to firing or activation of the stapler. Once the clinician has determined that the jaw members are clamping the tissue in a desired position, then the surgical stapler can be fired by the clinician to create an incision in the tissue and at the same time staple tissue surrounding the incision. This simultaneous severing/stapling action avoids complications that often arise when the severing and stapling operations are performed sequentially and/or with different surgical tools (i.e., one device is used to sever the tissue, and then another device is used to staple the tissue).

In general, application of various kinds of energy in association with closing a tissue incision can promote healing, reduce the possibility of infection, and/or promote proper sealing of the incision. International patent application WO03/057058, from which the preamble of claim 1 emanates, discloses surgical severing/stapling instrument having a heating element for cauterization of tissue. If assisted by the cauterizing action of electrical energy, for example, many surgical staplers could achieve better surgical results with respect to enhanced healing, improved infection resistance, and improved sealing of tissue incisions. However, the structure of many conventional surgical staplers, and the medical procedures in which they are employed, do not adequately leverage the beneficial effects of applying electrical energy to severed / stapled tissue.

In view of the foregoing, there is a need for improved electrosurgical instruments and electrical energy transmission systems than can more effectively and efficiently promote closure, treatment, and healing of tissue incisions created during procedures involving surgical staplers.

### SUMMARY

The invention provides an electrical energy transmission system structured for use with a surgical severing/stapling instrument including a removably connectable disposable severing/stapling unit as claimed herein after.

The invention further provides a surgical severing/stapling instrument having an electrical energy transmission system as claimed hereinafter.

### BRIEF DESCRIPTION OF THE FIGURES

The accompanying drawings, which are incorporated into and constitute a part of this specification, illustrate embodiments of the invention. Together with the description of the embodiments provided herein, the drawings serve to explain the principles of the invention for those skilled in the art.

Figure 1 includes a three-dimensional view of a surgical instrument provided in accordance with various embodiments of the invention;

Figure 2 includes a disassembled view of portions of a surgical instrument provided in accordance with various embodiments of the invention;

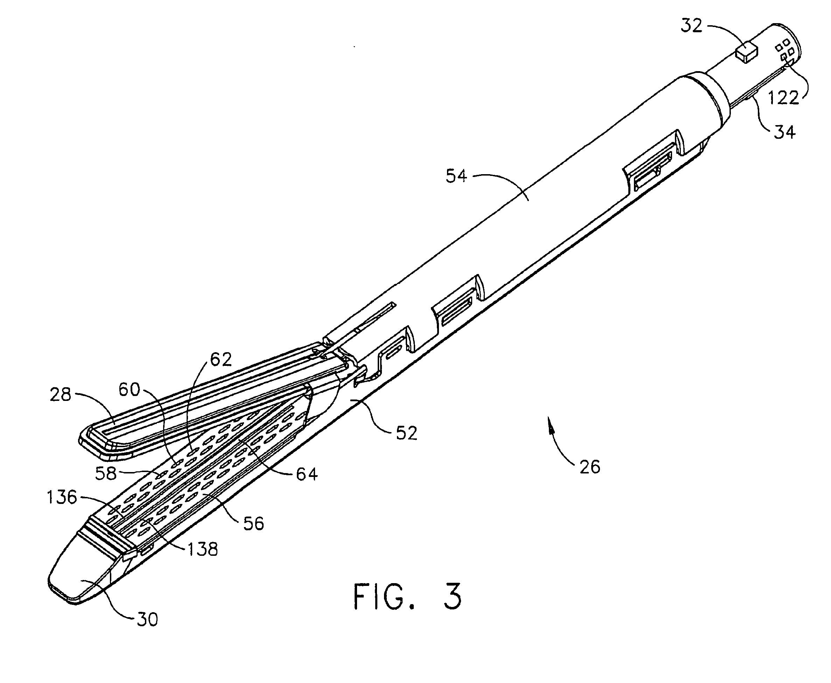

Figure 3 includes a three-dimensional top view of a disposable severing/stapling unit provided in accordance with various embodiments of the invention;

Figure 4 includes a three-dimensional bottom view of a disposable severing/stapling unit provided in accordance with various embodiments of the invention;

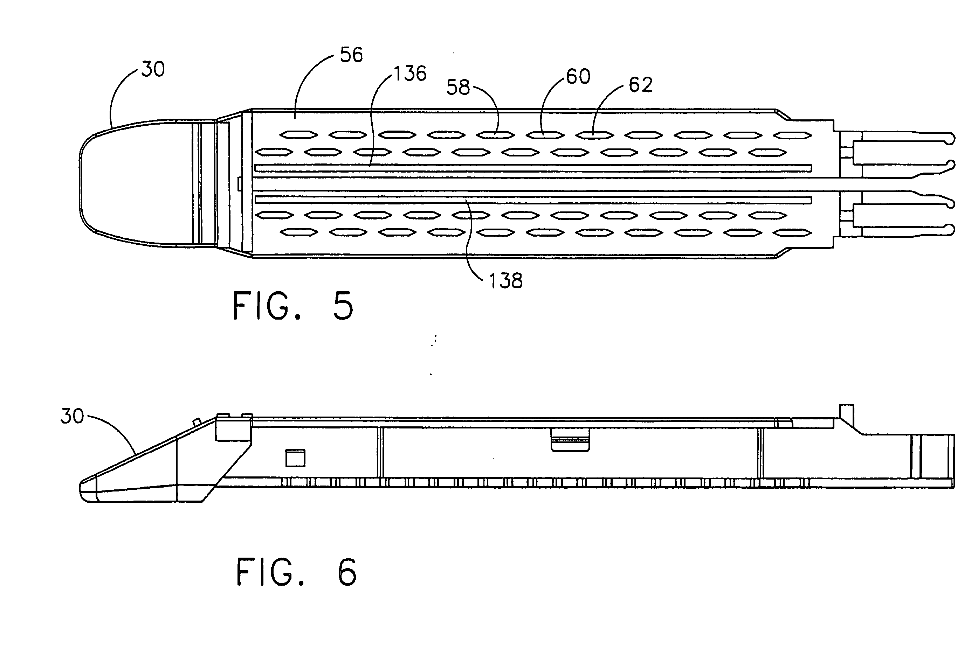

Figure 5 includes a top view of a staple cartridge assembly provided in accordance with various embodiments of the invention;

Figure 6 includes a side view of the cartridge assembly of Figure 5;

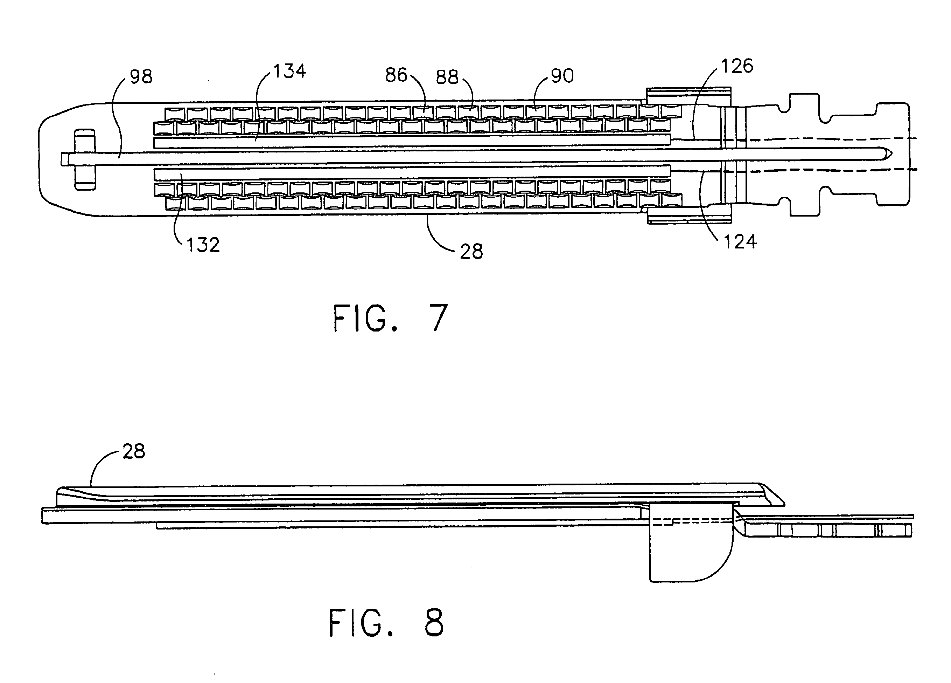

Figure 7 includes a bottom view of an anvil assembly provided in accordance with various embodiments of the invention; and,

Figure 8 includes a side view of the anvil assembly of Figure 7.

### DESCRIPTION

As applied herein, the term "tissue" may include a variety of human or animal tissues, membranes, or other organic substrates. The term "tissue" may also include any substance, substrate, or composition of matter capable of being severed, stapled or otherwise manipulated by the various embodiments of surgical stapling/severing instruments described herein.

With general reference to the figures, in association with various embodiments of the invention, a surgical severing/stapling instrument 10 including an electrical energy transmission system may be provided. The instrument 10 may include a handle portion 20 connected to an implement portion 22. The implement portion 22 includes a shaft 24 which extends distally from the handle portion 20 and which can be removably connected to a disposable severing/stapling unit 26. The disposable severing/stapling unit 26 may include a distally positioned anvil assembly 28 which is pivotally connected in the disposable unit 26 for opening / closing movement relative to a staple cartridge assembly 30. It can be seen that the spacing between the anvil assembly 28 and the staple cartridge assembly 30 may be configured to promote effective clamping, stapling and severing of tissue during use of the surgical instrument 10 by a clinician, for example, in a medical procedure.

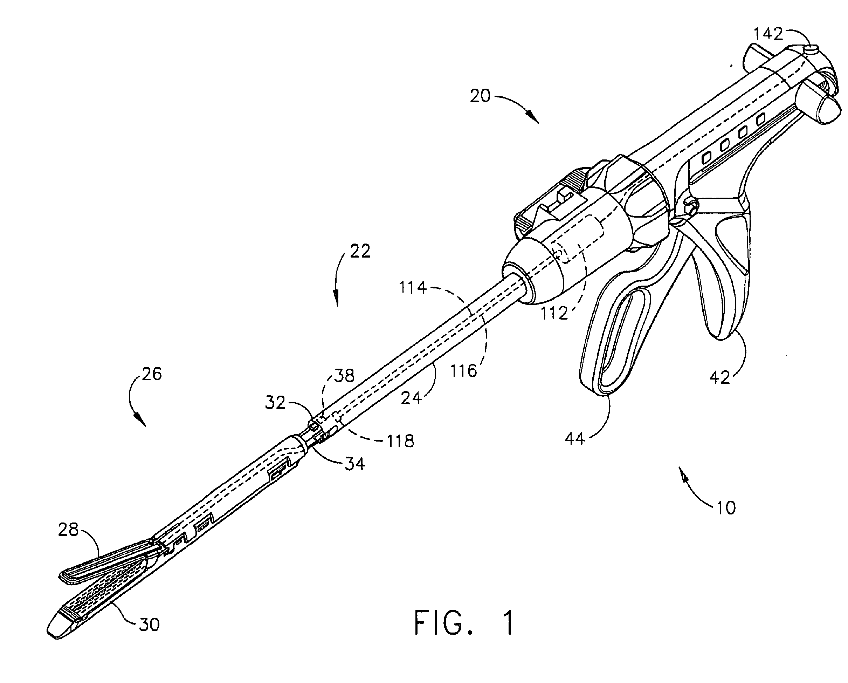

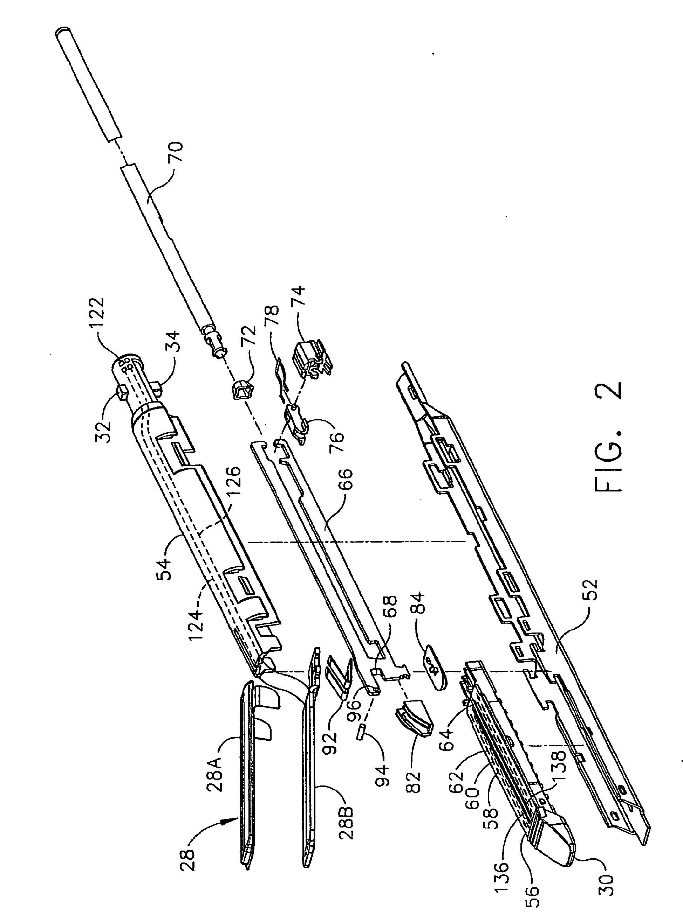

The removable connection between the disposable unit 26 and the shaft 24 may be accomplished by use of one or more push rods 32, 34 extending outwardly from the proximal end of the disposable severing/stapling unit 26. The push rods 32, 34 may be normally resiliently biased (e.g., by action of a conventional spring configuration) in the outward position (as shown in Figure 2, for example) after the disposable unit 26 is removed from the shaft 24 of the implement portion 22. In association with connection of the disposable unit 26 to the shaft 24, the push rods 32, 34 may be received in push rod depressions (such as illustrative depression 38, shown in Figure 1, for example) to promote locking of the disposable unit 26 in position with respect to the shaft 24 of the implement portion 22. It can be seen that the push rod 32, 34 configuration permits ready removal of the disposable unit 26, especially for applications wherein a new disposable unit 26 may be desirable for each successive firing of the instrument 10. The push rod 32, 34 configuration also resists unwanted dislodgement of the disposable unit 26 from the shaft 24 during use and operation of the instrument 10.

The handle portion 20 of the instrument 10 may include a grip 42 toward which a closure trigger 44 may be pivotally drawn by manual pressure applied by a clinician, for example, to cause clamping or closing of the anvil assembly 28 toward the cartridge assembly 30 of the disposable unit 26. In operations involving the surgical instrument 10, tissue may be clamped by the closing of the anvil assembly 28 toward the cartridge assembly 30. The handle portion 20 may also include a firing trigger or other functionality that causes the instrument 10, once the anvil assembly 28 is closed toward the cartridge assembly 30, to substantially simultaneously staple and sever tissue clamped in the disposable unit 26.

In various embodiments, the disposable unit 26 may include a lower channel 52 and an upper cover 54. The staple cartridge assembly 30 may be received within a distal end of the lower channel 42, as shown in Figure 2. In addition, a staple cartridge 56 may be positioned in the staple cartridge assembly 30. The staple cartridge 56 may have multiple staple holes (such as illustratively representative staple holes 58, 60, 62) formed therein and through which multiple staples (not shown) may be driven to staple severed tissue when the instrument 10 is fired.

The staple cartridge assembly 30 may also be configured with a longitudinal slot 64 that permits a knife shaft 66 having a distally positioned severing edge 68 to travel through the cartridge assembly 30. In operation, when the instrument 10 is fired, the knife shaft 66 and its severing edge 68 are moved along a longitudinal axis of the cartridge assembly 30 through the slot 64 by the action of a firing rod 70 cooperatively associated with the knife shaft 66. The cooperative interaction of the firing rod 70 with the knife shaft 66 may be achieved through use of an adapter 72 mounted on the distal end of the firing rod 70. The adapter 72 may be structured for connection to a drive block 74 that is connected to the knife shaft 66 with a locking member 76. The drive block 74 may be secured to the locking member 76 by use of a retaining clip 78, as shown in Figure 2. It can be seen that urging the severing edge 68 of the knife shaft 66 through the cartridge assembly 30 with the firing rod 70 functions to sever tissue clamped between the anvil assembly 28 and the cartridge assembly 30. In addition, a sled 82 may be positioned adjacent to a distal end of the knife shaft 66 to promote movement of the knife shaft 66 through the slot 64 of the cartridge assembly 30 and any tissue clamped between the cartridge assembly 30 and the anvil assembly 28. A mounting plate 84 may also be attached to a lower portion of the knife shaft 66 to promote stability and movement of the knife shaft 66 as it travels through the lower channel 52.

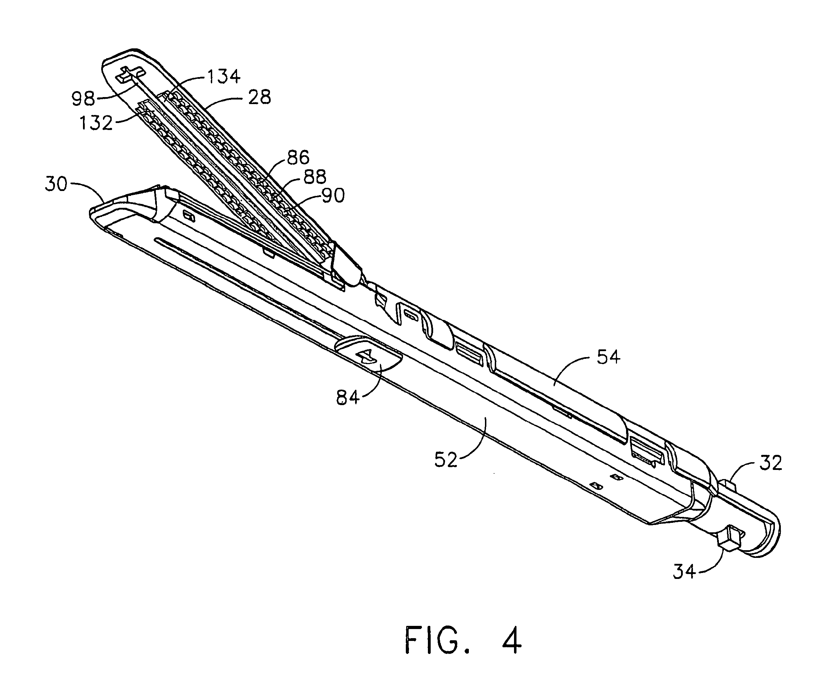

In various embodiments, the anvil assembly 28 may include at least two component parts: an upper plate 28A structured for attachment (such as by frictional fit) to a lower plate 28B. The lower plate 28B of the anvil assembly 28 may include a plurality of staple receiving depressions (such as illustratively representative depressions 86, 88, 90 shown in Figure 4, for example) formed therein which are structured for corresponding alignment with the staple holes 58, 60, 62. The staple receiving depressions 86, 88, 90 receive staples from the staple cartridge 56 that are driven through severed tissue by the instrument 10. A clip spring 92 may be positioned between a proximal portion of the lower plate 28B of the anvil assembly 28 and a distal portion of the knife shaft 66. The clip spring 92 may be structured with a resilient bias that promotes positioning of the anvil assembly 28 in a normally open position (i.e., a pre-firing position) relative to the staple cartridge assembly 30. Also, a pin 94 may be received in a hole 96 formed in a distal portion of the knife shaft 66 that can be structured for operative association with a slot 98 formed along a longitudinal axis of the anvil assembly 28. In operation of the instrument 10, the anvil assembly 28 can be moved toward the cartridge assembly 30, such as to clamp or position tissue therein, by action of the pin 94 as it initiates travel through the slot 98.

Examples of typical surgical severing/stapling instruments that are functionally and structurally analogous to, and that may be provided in association with, embodiments of the present invention are disclosed in a United States patent to Yates et al. entitled, "Electrosurgical Device and Method" (U.S. Pat. No. 5,833,690, issued on November 10, 1998).

In various embodiments, an energy transmission system may be provided in association with the surgical instrument 10 to promote closure and healing of tissue incisions created by the severing/stapling action of the instrument 10. The handle portion 20 may include an electrical power source 112 powered by a conventional battery or other suitable source of energy. The electrical power source 112 may be configured to generate and supply radio frequency (RF) energy, for example, that can be delivered by an electrical connection (such as wires 114, 116) to a first set of electrical contacts 118 positioned at the distal end of the shaft 24. It can be appreciated by those skilled in the art that characteristics of the RF energy such as frequency, current, voltage and other characteristics, may be selected to promote safe and effective application of the RF energy to severed tissue.

The disposable severing/stapling unit 26 may include a second set of electrical contacts 122 structured and positioned on the proximal end of the disposable unit 26 to complete an electrical circuit with the first set of contacts 118 when the disposable unit 26 is removably connected to the shaft 24 for use during operation of the instrument 10. The second set of contacts 122 may be electrically connected (such as by wires 124, 126) to a first set of electrodes 132, 134 positioned generally adjacent to rows of the multiple staple receiving depressions 86, 88, 90 formed in the anvil assembly 28. The second set of contacts 122 may also be electrically connected to at least a second set of electrodes 136, 138 positioned generally adjacent to rows of the multiple staple holes 58, 60, 62 formed in the staple cartridge 56 of the cartridge assembly 30. The electrodes 132, 134, 136, 138 may be comprised of any electrically conductive material such as copper, aluminum, silver, or other compositions of matter suitable for transferring electrical energy from the electrodes 132, 134, 136, 138 to severed tissue. It can be appreciated that conventional insulating structures and techniques may be employed to promote electrical isolation of the conductive components described herein for safe and effective transmission of electrical energy to the severed tissue.

Those skilled in the art will appreciate that either set of electrodes 132, 134, 136, 138, or individual electrodes 132, 134, 136, 138 within each of the sets, may be charged negatively or positively as desired to complete an electrical circuit capable of delivering electrical energy to severed tissue. For example, the first set of electrodes 132, 134 in the anvil assembly 28 may be charged positively; and the second set of electrodes 136, 138 in the staple cartridge assembly 30 may be charged negatively. In certain embodiments, the electrodes 132, 134, 136, 138, which are shown as single, continuous components in the figures for convenience of disclosure, may be separated into two or more separate components and appropriately electrically charged and electrically connected for transmission of electrical energy. In another example, one or more of the electrodes 132, 134, 136, 138 may be divided into two separate components: one component of the electrodes 132, 134, 136, 138 may be positively charged and another component of the electrodes 132, 134, 136, 138 may be negatively charged to create a current path in an electrical circuit for transmission of electrical energy between the two components. In certain embodiments, electrodes or electrode components may be positioned in specific predetermined locations on the anvil assembly 28 and/or the cartridge assembly 30 to facilitate focusing electrical energy on only certain portions of severed tissue.

In various embodiments, the disposable severing/stapling unit 26 may be provided with one or both of the electrodes 132, 134 positioned on the anvil assembly 28, but with no corresponding electrodes positioned on the cartridge assembly 30. In such embodiments, the electrodes 132, 134 on the anvil assembly 28 may be electrically connected to the electrical power source 112 to receive transmitted electrical current; and, at least a portion of the cartridge assembly 30 (e.g., a portion or portions of the staple cartridge 56) may be electrically grounded through a connection with the ground of the electrical power source 112, for example, or through another suitable ground connection. Thus, one or both of the electrodes 132, 134 may be positively charged, and at least a portion of the cartridge assembly 30 may be electrically grounded. It can be seen that these configurations create a circuit that facilitates the flow of electrical current from the electrodes 132, 134, through portions of severed/stapled tissue clamped between the anvil assembly 28 and the cartridge assembly 30, to the grounded cartridge assembly 30.

In various embodiments, the disposable severing/stapling unit 26 may be provided with one or both of the electrodes 136, 138 positioned on the cartridge assembly 30, but with no corresponding electrodes positioned on the anvil assembly 30. In such embodiments, the electrodes 136, 138 on the cartridge assembly 30 may be electrically connected to the electrical power source 112 to receive transmitted electrical current; and, at least a portion of the anvil assembly 28 may be electrically grounded through a connection with the ground of the electrical power source 112, for example, or through another suitable ground connection. Thus, one or both of the electrodes 136, 138 may be positively charged, and at least a portion of the anvil assembly 28 may be electrically grounded. It can be seen that these configurations create a circuit that facilitates the flow of electrical current from the electrodes 136, 138, through portions of severed/stapled tissue clamped between the anvil assembly 28 and the cartridge assembly 30, to the grounded anvil assembly 28.

Electrical energy (e.g., RF electrical energy) can be employed in connection with operation of the surgical instrument 10 to cauterize or otherwise promote closure or sealing of severed tissue. The electrical power source 112 is activated by a button or trigger 142 to manually cause current to flow through the energy transmission system to the electrodes 132, 134, 136, 138. According to the invention the electrical power source 112 is configured to activate and generate electrical energy automatically in association with firing the instrument 10. For example, in association with firing the instrument 10 to sever/staple tissue, the electrical power source 112 may be automatically activated to transmit RF energy through one or more of the electrodes 132, 134, 136, 138 of the energy transmission system to cauterize or otherwise promote closure and sealing of tissue severed and stapled by action of the instrument 10.

It will be appreciated that the terms "proximal" and "distal" may be used herein as convenient terms of relative orientation, such as with reference to a clinician gripping a handle of an instrument. For example, the disposable unit 26 may be considered "distal" with respect to the "proximal" handle portion 20 (see, e.g., Figure 1). It will be further appreciated that, for convenience and clarity of disclosure, spatially paired terms of relative orientation such as "top" and "bottom"; "upper" and "lower"; or "downward" and "upward" may be used herein with respect to the drawings. Those skilled in the art will appreciate, however, that surgical instruments may be used in many orientations and positions, and such terms are not intended to be limiting and absolute.

The examples presented herein are intended to illustrate potential and specific implementations of the present invention for those skilled in the art. No particular aspect or aspects of the examples included herein are necessarily intended to limit the scope of the present invention.

It is to be understood that the figures and descriptions of the present invention have been simplified to illustrate elements that are relevant for a clear understanding of the present invention, while eliminating, for purposes of clarity, other elements. Those of ordinary skill in the art will recognize, however, that these and other elements may be desirable in a typical computer system or database system. However, because such elements are well known in the art and because they do not facilitate a better understanding of the present invention, a discussion of such elements may not be provided herein.

In various embodiments of the present invention disclosed herein, a single component may be replaced by multiple components, and multiple components may be replaced by a single component, to perform a given function or functions. Except where such substitution would not be operative to practice embodiments of the present invention, such substitution is within the scope of the present invention.

While the present invention has been illustrated by description of several embodiments and while the illustrative embodiments have been described in considerable detail, it is not the intention of the applicant to restrict or in any way limit the scope of the appended claims to such detail. Additional advantages and modifications may readily appear to those skilled in the art. The present invention has been discussed in terms of endoscopic procedures and apparatus. However, use herein of terms such as "endoscopic" should not be construed to limit the present invention to a surgical stapling and severing instrument for use only in conjunction with an endoscopic tube (i.e., trocar). On the contrary, it is believed that surgical instruments structured in accordance with the present invention may find use in many surgical procedures, including but not limited to laparoscopic procedures and open procedures. Moreover, the unique and novel aspects of the embodiments of the present invention may find utility when used in connection with other forms of stapling apparatuses without departing from the scope of the present invention.

## Claims

1. An electrical energy transmission system structured for use with a surgical severing/stapling instrument (10) including a removably connectable disposable severing/stapling unit (26), the energy transmission system comprising:
an electrical power source (112); and,
at least one electrode (132, 134, 136, 138) positioned on at least one of an anvil assembly (28) or a cartridge assembly (30) of the disposable severing/stapling unit, the assembly electrode being configured to receive electrical power from the electrical power source when the disposable severing/stapling unit is removably connected to the surgical instrument;
**characterised in that** the electrical power source is configured to activate and generate electrical energy automatically in association with firing of the surgical instrument.

2. The energy transmission system of Claim 1, wherein the electrical power source (112) includes a radio frequency electrical power source.

3. The energy transmission system of Claim 1, further comprising the disposable severing/stapling unit (26) having a set of electrical contacts (122) positioned thereon to create an electrical circuit with a set of electrical contacts (118) positioned on a shaft (24) of the surgical instrument when the disposable unit is removably connected to the shaft.

4. The energy transmission system of Claim 1, wherein the assembly electrode includes an anvil assembly electrode (132, 134) positioned generally adjacent to a plurality of staple receiving depressions formed in the anvil assembly (28).

5. The energy transmission system of Claim 1, wherein the assembly electrode includes a cartridge assembly electrode (136, 138) positioned on a staple cartridge received within the cartridge assembly (30).

6. The energy transmission system of Claim 5, further comprising the cartridge assembly electrode (136, 138) being positioned generally adjacent to a plurality of staple holes formed in the staple cartridge (30).

7. The energy transmission system of Claim 1, wherein at least a portion of one of the anvil assembly or the cartridge assembly is electrically grounded.

8. A surgical severing/stapling instrument having an electrical energy transmission system of any preceding claim, the surgical instrument further comprising:
a handle portion (20) including the electrical power source therein;
an implement portion (22) connected to the handle portion, the implement portion including a shaft (24) structured for removably connecting the disposable severing/stapling unit (26) thereto.

## Patentansprüche

1. Ein elektrisches Energieübertragungssystem, das zur Verwendung mit einem chirurgischen Abtrenn-/Klammerinstrument (10) strukturiert ist, das eine entfernbare verbindbare Einweg-Abtrenn-/Klammereinheit (26) aufweist, wobei das Energieübertragungssystem umfasst:
eine elektrische Stromquelle (112); und
wenigstens eine Elektrode (132, 134, 136, 138), die auf einer Ambossanordnung (28) und/oder einer Patronenanordnung (30) der Einweg-Abtrenn-/Klammereinheit positioniert ist, wobei die Anordnungselektrode konfiguriert ist, um elektrischen Strom von der elektrischen Stromquelle zu empfangen, wenn die Einweg-Abtrenn-/Klammereinheit mit dem chirurgischen Instrument entfernbar verbunden ist;
**dadurch gekennzeichnet, dass** die elektrische Stromquelle konfiguriert ist, um in Verbindung mit dem Auslösen des chirurgischen Instruments elektrische Energie automatisch zu aktivieren und generieren.

2. Energieübertragungssystem nach Anspruch 1, **dadurch gekennzeichnet, dass** die elektrische Stromquelle (112) eine elektrische Hochfrequenzstromquelle aufweist.

3. Energieübertragungssystem nach Anspruch 1, ferner umfassend, dass die Einweg-Abbrenn-/Klammereinheit (26) eine Gruppe von elektrischen Kontakten (122) aufweist, die auf derselben positioniert sind, um einen elektrischen Stromkreis mit einer Gruppe von elektrischen Kontakten (118) zu erzeugen, die auf einem Schaft (24) des chirurgischen Instruments positioniert sind, wenn die Einwegeinheit mit dem Schaft entfernbar verbunden ist.

4. Energieübertragungssystem nach Anspruch 1, **dadurch gekennzeichnet, dass** die Anordnungselektrode eine Ambossanordnungselektrode (132, 134) aufweist, die im allgemeinen neben einer Vielzahl von Klammern positioniert ist, die Vertiefungen, die in der Ambossanordnung (28) gebildet werden, erhalten.

5. Energieübertragungssystem nach Anspruch 1, **dadurch gekennzeichnet, dass** die Anordnungselektrode eine Patronenanordnungselektrode (136, 138) aufweist, die auf einer Klammerpatrone positioniert ist, die innerhalb in der Einsatzanordnung (30) aufgenommen ist.

6. Energieübertragungssystem nach Anspruch 5, ferner umfassend, dass die Patronenanordnungselektrode (136, 138) im allgemeinen neben einer Vielzahl von Klammerlöchem positioniert ist, die in der Klammerpatrone (30) gebildet werden.

7. Energieübertragungssystem nach Anspruch 1, **dadurch gekennzeichnet, dass** ein Abschnitt der Ambossanordnung und/oder der Einsatzanordnung elektrisch geerdet ist.

8. Chirurgisches Abtrenn-/Klammerinstrument, das ein elektrisches Energieübertragungssystem nach einem der vorangehenden Ansprüche aufweist, wobei das chirurgische Instrument ferner umfasst:
einen Griffabschnitt (20), in dem sich die elektrische Stromquelle befindet;
einen Werkzeugabschnitt (22), der mit dem Griffabschnitt verbunden ist, wobei der Werkzeugabschnitt einen Schaft (24) aufweist, der strukturiert ist, um die Einweg-Abtrenn-/Klammereinheit (26) damit lösbar zu verbinden,

## Revendications

1. Système de transmission d'énergie électrique structuré pour être utilisé avec un instrument chirurgical de séparation/agrafage (10) comprenant une unité de séparation/agrafage jetable connectable de manière amovible (26), le système de transmission d'énergie comprenant :
➢ une source de puissance électrique (112) ; et
➢ au moins une électrode (132, 134, 136, 138) positionnée sur au moins l'un d'un ensemble d'enclume (28) et d'un ensemble de cartouche (30) de l'unité de séparation/agrafage jetable, l'électrode d'ensemble étant configurée pour recevoir une puissance électrique de la source de puissance électrique lorsque l'unité de séparation/agrafage jetable est connectée de façon amovible à l'instrument chirurgical ;
**caractérisé en ce que** la source de puissance électrique est configurée pour activer et générer de l'énergie électrique automatiquement en association avec l'allumage de l'instrument chirurgical.

2. Système de transmission d'énergie selon la revendication 1, dans lequel la source de puissance électrique (112) comprend une source de puissance électrique à radiofréquences.

3. Système de transmission d'énergie selon la revendication 1, comprenant en outre l'unité de séparation/ agrafage jetable (26) ayant un jeu de contacts électriques (122) positionnés sur celle-ci pour créer un circuit électrique avec un jeu de contacts électriques (118) positionnés sur une tige (24) de l'instrument chirurgical lorsque l'unité jetable est connectée de façon amovible à la tige.

4. Système de transmission d'énergie selon la revendication 1, dans lequel l'électrode d'ensemble comprend une électrode d'ensemble d'enclume (132, 134) positionnée globalement adjacente à une pluralité de creux de réception d'agrafes formés dans l'ensemble d'enclume (28).

5. Système de transmission d'énergie selon la revendication 1, dans lequel l'électrode d'ensemble comprend une électrode d'ensemble de cartouche (136, 138) positionnée sur une cartouche d'agrafes reçue à l'intérieur de l'ensemble de cartouche (30).

6. Système de transmission d'énergie selon la revendication 5, comprenant en outre l'électrode d'ensemble de cartouche (136, 138) positionnée globalement adjacente à une pluralité de trous d'agrafes formés dans la cartouche d'agrafes (30).

7. Système de transmission d'énergie selon la revendication 1, dans lequel au moins une partie de l'un de l'ensemble d'enclume et de l'ensemble de cartouche est reliée à la terre électriquement.

8. Instrument chirurgical de séparation/agrafage ayant un système de transmission d'énergie électrique selon l'une quelconque des revendications précédentes, l'instrument chirurgical comprenant en outre :
➢ une partie de poignée (20) intégrant dans celle-ci la source de puissance électrique ;
➢ une partie d'accessoire (22) reliée à la partie de poignée, la partie d'accessoire comprenant une tige (24) structurée pour connecter de façon amovible l'unité de séparation/agrafage jetable (26) à celle-ci.
